# EUROPEAN PATENT SPECIFICATION

(11) **EP 4 348 234 B1**
(45) Date of publication and mention of the grant of the patent: **08.04.2026**
(21) Application number: 22743956.9
(22) Date of filing: 01.06.2022
(51) Int. Cl.: G01N 27/447, A61K 38/00, C07K 1/26, G01N 33/68

(54) **MICROCHIP CAPILLARY ELECTROPHORESIS ASSAYS AND REAGENTS**
MIKRO-CHIP-KAPILLARELEKTROPHORESE-ASSAYS UND REAGENZIEN
ANALYSES ET RÉACTIFS POUR ÉLECTROPHORÈSE CAPILLAIRE SUR MICROPUCE

(30) Priority: 01.06.2021 US 202117335756; 06.07.2021 US 202117368377
(43) Date of publication of application: 10.04.2024
(60) Divisional application: 26156454.6
(73) Proprietor: Regeneron Pharmaceuticals, Inc., Tarrytown, NY 10591 (US)
(72) Inventor: RIEHLMAN, Timothy, Tarrytown, NY 10591 (US); CARREAU, Gabriel, Tarrytown, NY 10591 (US); SCHNEIDERHEINZE, Jeffrey, Tarrytown, NY 10591 (US); NALL, Nicole, M., Tarrytown, NY 10591 (US)
(74) Representative: Reddie & Grose LLP
(86) International application number: PCT/US2022/031738
(87) International publication number: WO 2022/256383

(56) References cited:
- US-A1- 2019 285 580
- AN PHILIP ET AL: "Automated high-throughput dense matrix protein folding screen using a liquid handling robot combined with microfluidic capillary electrophoresis", PROTEIN EXPRESSION AND PURIFICATION, ACADEMIC PRESS, SAN DIEGO, CA, vol. 120, 8 December 2015 (2015-12-08), pages 138 - 147, XP029393679, ISSN: 1046-5928, DOI: 10.1016/J.PEP.2015.11.015

## Description

### CROSS-REFERENCE TO RELATED APPLICATIONS

This application claims benefit of and priority to U.S. Patent Application No. 17/335,756 filed on 1 June 2021 and U.S. Patent Application No. 17/368,377 filed on 6 July 2021.

### TECHNICAL FIELD OF THE INVENTION

Aspects of the invention are generally directed to the field of capillary electrophoresis, in particular to microchip capillary electrophoresis.

### BACKGROUND OF THE INVENTION

Implementation of robust, reproducible, user-friendly technology is critical to meet the testing demands of biological products in today's Quality Control (QC) laboratories. Upgrades in technology are necessary to facilitate increased output, while continuing to generate quality analytical data and attempting to minimize the number of invalid test results and instrumentrelated investigations. While electrophoresis has historically been used in QC for product purity and fragmentation analysis, the methodology has transitioned from gel-based, to capillary-based, and more recently, to the microchip. Microchip Capillary Electrophoresis (MCE) allows for dramatically reduced sample analysis times, while maintaining the performance and reproducibility standards required for QC analysis (Ouimet, C., et al., Expert Opin Drug Discov., 12(2): 213-224 (2017)).

Although MCE has emerged as a promising technique with growing use in the pharmaceutical industry for characterizing biopharmaceuticals, quality control, and for drug discovery, it can be prone to assay interferences.

Thus, it is an object of the invention to provide improved MCE assays and compositions that reduce assay interferences.

It is another object of the invention to provide MCE assays and compositions for improving detection of impurities in a protein drug product.

US 2019/285580 A1 discloses MCE assays and reagents to assess purity and to identify impurities in protein drug product samples, and methods for analyzing analytes in a protein drug sample. The aqueous electrophoresis sample buffer used for identifying contaminants in protein samples comprises 2-iodoacetamide, lithium dodecyl sulphate and sodium phosphate, and has a specific pH.

### SUMMARY OF THE INVENTION

MCE assays and reagents to assess purity and to identify impurities in protein drug product samples are provided. Methods for analyzing analytes in a protein drug sample are provided. Preferred protein drugs include but are not limited to recombinant proteins such as antibodies and antigen binding fragments thereof, and fusion proteins. The assays employ MCE techniques to separate, identify, and quantify protein product and impurities in the protein product. Impurities include, but are not limited to protein aggregates, protein fragments, protein multimers, and assay contaminants. Reducing and non-reducing buffers are also provided. In some embodiments, the MCE assays and reagents provided herein may be used in the analysis and purity testing of Anti-SARS-CoV-2 products, such as therapeutic protein products including REGEN-COV^{™} (casirivimab and imdevimab).

According to the invention, the non-reducing aqueous electrophoresis sample buffer contains 2-iodoacetamide (IAM). In one example which is excluded from the claimed subject-matter, the non-reducing aqueous electrophoresis sample buffer contains an alkylating agent, 155 to 175 mM 2-iodoacetamide; 0.50 to 1.5% lithium dodecyl sulfate; and 65 to 95 mM sodium phosphate, wherein the aqueous electrophoresis sample buffer has a pH of less than 7. In an embodiment, the pH of the buffer is 6. In another embodiment, the aqueous buffer contains 166 mM 2-iodoacetamide, 0.81% lithium dodecyl sulfate, and 81 mM sodium phosphate.

In another embodiment, the non-reducing aqueous electrophoresis sample buffer contains 155 to 200 mM 2-iodoacetamide; 0.50 to 1.5% lithium dodecyl sulfate; and 60 to 95 mM sodium phosphate, wherein the aqueous electrophoresis sample buffer has a pH of less than or equal to 8. In an embodiment, the pH of the buffer is 8. In yet another embodiment, the aqueous buffer contains 200 mM 2-iodoacetamide, 1.2% lithium dodecyl sulfate, and 60 mM sodium phosphate.

A reducing buffer is also provided. In one embodiment, the reducing buffer is an aqueous electrophoresis sample buffer containing 0.5 to 1.5% lithium dodecyl sulfate, 55 to 75 mM sodium phosphate, and a reducing agent, wherein the aqueous electrophoresis sample buffer has a pH greater than 8. In an embodiment, the pH of the buffer is 9. In an example which is excluded from the claimed subject-matter, the reducing buffer contains 135 to 155 mM dithiothreitol.

In another embodiment, the reducing buffer is an aqueous electrophoresis sample buffer containing 0.5 to 1.5% lithium dodecyl sulfate, 45 to 75 mM sodium phosphate, and a reducing agent, wherein the aqueous electrophoresis sample buffer has a pH of 8 or greater. In an embodiment, the pH of the buffer is 8. In one embodiment, the reducing buffer contains 80 to 155 mM dithiothreitol. Still another embodiment provides a reducing buffer containing 1.2 % lithium dodecyl sulfate, 60 mM sodium phosphate, and 80 mM dithiothreitol.

HEPES based buffers, although not part of the claimed subject-matter, can also be used with the disclosed methods. One embodiment provides a non-reducing HEPES based aqueous electrophoresis sample buffer containing an alkylating agent, for example 55 to 75 mM 2-iodoacetamide; 0.1 to 1.0 % lithium dodecyl sulfate; 5 to 85 mM HEPES, and 5 to 115 mM sodium chloride, wherein the aqueous electrophoresis sample buffer has a pH of less than 9. In another embodiment, the pH of the buffer is 8. In still another embodiment, the aqueous buffer contains 66.4 mM 2-iodoacetamide, 0.32% lithium dodecyl sulfate, 16.2 mM HEPES, and 48.6 mM sodium chloride.

Another embodiment provides a reducing HEPES based aqueous electrophoresis sample buffer containing 0.05 to 0.75 % lithium dodecyl sulfate, 5 mM to 115 mM sodium chloride, 5 mM to 115 mM HEPES, and a reducing agent, wherein the aqueous electrophoresis sample buffer has a pH greater than 7. In an embodiment, the pH of the buffer is 8. In one embodiment, the reducing buffer contains 35 to 50 mM dithiothreitol. Still another embodiment provides a reducing buffer containing 0.28 % lithium dodecyl sulfate, 41.5 mM sodium chloride, 13.8 mM HEPES, and 42.5 mM dithiothreitol.

The disclosure provides a non-reducing MCE method for identifying contaminants or impurities in a protein drug sample including the steps of adding the protein sample to a non-reducing buffer discussed above to form a buffered protein drug sample. In an example which is excluded from the claimed subject-matter, the buffered protein drug sample is heated to between 65 to 85 °C for 5 to 15 minutes to form a denatured buffered protein drug sample. For example, the buffered protein drug sample is heated to 70 °C for 10 min.

In an embodiment, the buffered protein drug sample is heated to 63 °C for 10 min. In another embodiment, the buffered protein drug sample is heated to 60 °C for 10 min.

In some embodiments, the protein drug sample is mixed with a detectable label and heated at 30 to 40 °C for 20 to 40 minutes or for 10 to 40 minutes to form a denatured labeled protein drug sample. In other embodiments, the protein drug sample is mixed with a detectable label and heated at 30 to 40 °C for 15 minutes to form a denatured labeled protein drug sample. A detectable label includes, but is not limited to Dyomics DY-631 NHS Ester. In some embodiments, the label is diluted by MilliQ purified water, and in other embodiments the label is diluted in a sodium phosphate buffer. In some examples, the detectable label is reconstituted with dimethyl sulfoxide (DMSO) followed by dilution with 200 mM Sodium Phosphate pH 7.2 buffer. Other detectable labels can be used, including other dyes, fluorophores, chromophores, mass tags, quantum dots and the like and those disclosed in US Patent No. 6,924,372. In an embodiment, the protein drug sample with added label is heated to 35 °C for 30 minutes. In another embodiment, the protein drug sample with added label is heated to 35 °C for 15 minutes. Excess label is optionally removed from the sample, for example by using a spin filter. In still another embodiment, excess label is not quenched. In still another embodiment, excess label is not removed.

The denatured labeled protein drug product is diluted and subjected to MCE to separate the diluted protein drug sample on a microchip capillary electrophoresis system to produce an electropherogram. In one embodiment the final concentration of a sample starting at 0.5 mg/ml that is then injected over the microchip is 9 µg/ml to MCE. In another embodiment, the final concentration of a sample starting at 0.2 mg/ml that is then injected over the microchip is 3.6 µg/ml to MCE. The electropherogram contains peaks corresponding to the protein drug product and impurities. The method concludes by identifying peaks in the electropherogram corresponding to the protein drug product, contaminants, and/or impurities.

Another embodiment provides a reducing MCE method for identifying contaminants or impurities in a protein drug sample. The method begins by adding the protein sample to any one of the reducing buffers described above to form a buffered protein drug sample. The protein drug sample is mixed with a detectable label and heated at 30 to 40 °C for 20 to 40 minutes to form a denatured labeled protein drug sample. In an embodiment, the protein drug sample with added label is heated to 35 °C for 30 minutes.

In other embodiments, the buffered protein drug sample is denatured by heating the buffered protein drug sample to 60 °C for 10 minutes to form a denatured protein drug sample. For example, the protein drug sample is mixed with a detectable label and heated at 30 to 40 °C for 15 minutes to form a denatured labeled protein drug sample. In another embodiment, the protein drug sample with added label is heated to 35 °C for 15 minutes.

Excess label is optionally removed from the sample, for example by using a spin filter. In still another embodiment, excess label is not quenched. In still another embodiment, excess label is not removed. An exemplary detectable label includes, but is not limited to Dyomics DY-631 NHS Ester. Other detectable labels that can be used include other dyes, fluorophores, chromophores, mass tags, quantum dots and the like and those disclosed in US Patent No. 6,924,372.

In one embodiment, the established assay range for sample concentration is from 0.4 mg/ml to 0.6 mg/ml, corresponding to a final concentration being analyzed of about 7 µg/ml to 11 µg/ml which is subjected to MCE analysis on a microchip capillary electrophoresis system to produce an electropherogram. In another embodiment, the established assay range for sample concentration is from 0.2 mg/ml to 0.6 mg/ml, corresponding to a final concentration being analyzed of about 3.6 µg/ml to 11 µg/ml. The method concludes by identifying peaks in the electropherogram corresponding to the protein drug product, contaminants, and/or impurities.

The invention is defined in the appended claims.

### BRIEF DESCRIPTION OF THE DRAWINGS

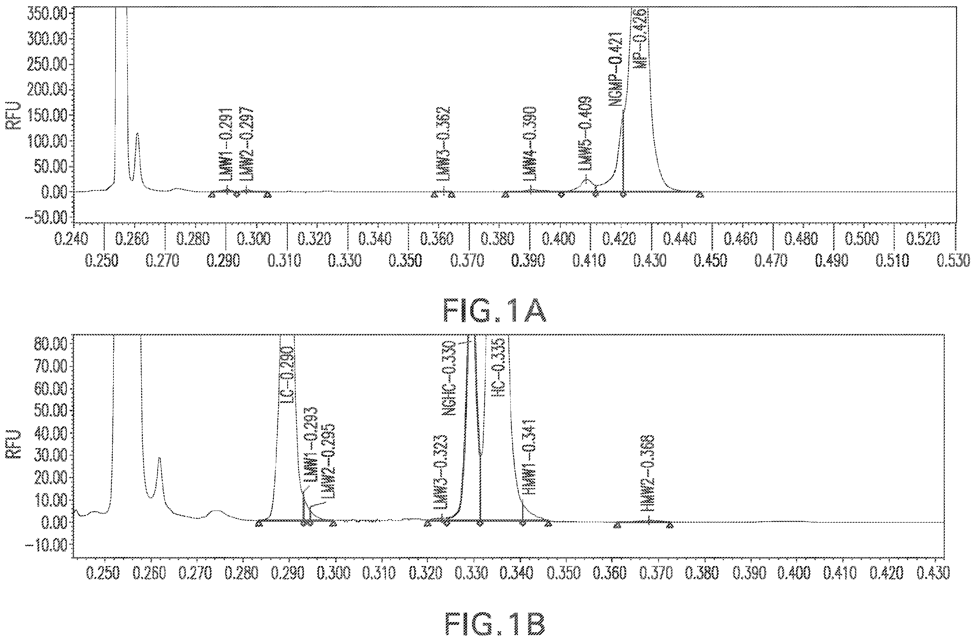

**Figure 1A** shows an electropherogram of a typical non-reduced sample analysis.
**Figure 1B** shows an electropherogram of a typical reduced sample analysis. The X-axis represents time in minutes, and the Y-axis represents the relative fluorescence units (RFU). Increased migration time corresponds to increased protein size.

### DETAILED DESCRIPTION OF THE INVENTION

### I. Definitions

The use of the terms "a," "an," "the," and similar referents in the context of describing the presently claimed invention (especially in the context of the claims) are to be construed to cover both the singular and the plural, unless otherwise indicated herein or clearly contradicted by context.

Recitation of ranges of values herein are merely intended to serve as a shorthand method of referring individually to each separate value falling within the range, unless otherwise indicated herein, and each separate value is incorporated into the specification as if it were individually recited herein.

Use of the term "about" is intended to describe values either above or below the stated value in a range of approx. +/- 10%; in other embodiments the values may range in value either above or below the stated value in a range of approx. +/- 5%; in other embodiments the values may range in value either above or below the stated value in a range of approx. +/- 2%; in other embodiments the values may range in value either above or below the stated value in a range of approx. +/- 1%. The preceding ranges are intended to be made clear by context, and no further limitation is implied. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any nonclaimed element as essential to the practice of the invention.

"Protein" refers to a molecule comprising two or more amino acid residues joined to each other by a peptide bond. Protein includes polypeptides and peptides and may also include modifications such as glycosylation, lipid attachment, sulfation, gamma-carboxylation of glutamic acid residues, alkylation, hydroxylation and ADP-ribosylation. Proteins can be of scientific or commercial interest, including protein-based drugs, and proteins include, among other things, enzymes, ligands, receptors, antibodies and chimeric or fusion proteins. Proteins are produced by various types of recombinant cells using well-known cell culture methods, and are generally introduced into the cell by genetic engineering techniques (e.g., such as a sequence encoding a chimeric protein, or a codon-optimized sequence, an intronless sequence, etc.) where it may reside as an episome or be integrated into the genome of the cell.

"Antibody" refers to an immunoglobulin molecule consisting of four polypeptide chains, two heavy (H) chains and two light (L) chains inter-connected by disulfide bonds. Each heavy chain has a heavy chain variable region (HCVR or VH) and a heavy chain constant region. The heavy chain constant region contains three domains, CH1, CH2 and CH3. Each light chain has a light chain variable region and a light chain constant region. The light chain constant region consists of one domain (CL). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The term "antibody" includes reference to both glycosylated and non-glycosylated immunoglobulins of any isotype or subclass. The term "antibody" includes antibody molecules prepared, expressed, created or isolated by recombinant means, such as antibodies isolated from a host cell transfected to express the antibody. The term antibody also includes bispecific antibody, which includes a heterotetrameric immunoglobulin that can bind to more than one different epitope. Bispecific antibodies are generally described in US Patent No. 8,586,713.

"Fc fusion proteins" comprise part or all of two or more proteins, one of which is an Fc portion of an immunoglobulin molecule, which are not otherwise found together in nature. Preparation of fusion proteins comprising certain heterologous polypeptides fused to various portions of antibody-derived polypeptides (including the Fc domain) has been described, e.g., by Ashkenazi et al., Proc. Natl. Acad. Sci USA, 88: 10535 (1991); Byrn et al., Nature 344:677 (1990); and Hollenbaugh et al., "Construction of Immunoglobulin Fusion Proteins", in Current Protocols in Immunology, Suppl. 4, pages 10.19.1 - 10.19.11 (1992). "Receptor Fc fusion proteins" comprise one or more extracellular domain(s) of a receptor coupled to an Fc moiety, which in some embodiments comprises a hinge region followed by a CH2 and CH3 domain of an immunoglobulin. In some embodiments, the Fc-fusion protein comprises two or more distinct receptor chains that bind to a one or more ligand(s). For example, an Fc-fusion protein is a trap, such as for example an IL-1 trap or VEGF trap.

The term "MCE" or "Microchip Capillary Electrophoresis" refers to microchip-based capillary electrophoresis (CE) separation of analytes.

### II. MCE Assays and Buffers

Methods for analyzing analytes in a protein drug sample are provided. Protein drugs include, but are not limited to antibodies and antigen binding fragments thereof, fusion proteins, and recombinant proteins. The assays employ MCE techniques to separate, identify, and quantify protein product and impurities in the protein product. Impurities include, but are not limited to protein aggregates, protein fragments, protein multimers, and assay contaminants. Reducing and non-reducing buffers are also provided.

Microchip Capillary Electrophoresis (MCE) provides analysis of protein purity and impurities suitable for in-process testing, lot release, and stability indication. Protein samples, such as casirivimab and imdevimab, were prepared by denaturing the sample through heating and addition of lithium dodecyl sulfate (LDS) with either an alkylating agent (non-reduced) or a reducing agent (reduced). Not intending to be bound by theory, this produces linear negatively charged protein-LDS polypeptide chains, which are then incubated with an amine ester dye to covalently label any free amine groups. Labeled samples may be injected onto a microchip where the application of a current separates the sample components according to mass to charge ratio. Detection can be performed by laser-induced fluorescence, producing an electropherogram. Analysis of the electropherogram provides the relative purity of the sample compared to the impurities present.

### A. Buffers

### 1. Non-reducing Buffers

One embodiment provides a non-reducing aqueous electrophoresis sample buffer containing 155 to 200 mM of 2-iodoacetamide; 0.50 to 1.5% lithium dodecyl sulfate; and 60 to 95 mM sodium phosphate, wherein the aqueous electrophoresis sample buffer has a pH of less than 7. In an embodiment, the pH of the buffer is 6.

Another embodiment provides a non-reducing aqueous electrophoresis sample buffer containing 155 to 200 mM of 2-iodoacetamide; 0.50 to 1.5% lithium dodecyl sulfate; and 60 to 95 mM sodium phosphate, wherein the aqueous electrophoresis sample buffer has a pH of less than 8. In another embodiment, the pH of the buffer is 8. In another embodiment, the aqueous buffer contains 200 mM 2-iodoacetamide, 1.2% lithium dodecyl sulfate, and 60 mM sodium phosphate.

All embodiments are with the proviso that the buffer is not an aqueous electrophoresis sample buffer containing 155 to 175 mM 2-iodoacetamide, 0.50 to 1.5% lithium dodecyl sulfate, and 60 to 95 mM sodium phosphate, and having a pH of less than 7.

### 2. Reducing Buffers

A reducing buffer is also provided. In one embodiment, the reducing buffer is an aqueous electrophoresis sample buffer containing 0.5 to 1.5% lithium dodecyl sulfate, 65 to 75 mM sodium phosphate, and a reducing agent, wherein the aqueous electrophoresis sample buffer has a pH greater than 8. In another embodiment, the reducing buffer contains 0.5 to 1.5% lithium dodecyl sulfate, 45 to 75 mM sodium phosphate, and a reducing agent, and the aqueous electrophoresis sample buffer has a pH of 8 or greater. In an embodiment, the pH of the buffer is 9. In another embodiment, the pH of the buffer is 8.

Reducing agents are known in the art. Exemplary reducing agents include but are not limited to dithiothreitol (DTT, CAS 3483-12-3), beta-mercaptoethanol (BME, 2BME, 2-ME, b-mer, CAS 60-24-2), 2-aminoethanethiol (2-MEA-HCl, also called cysteamine-HCl, CAS 156-57-0), Tris (2-carboxyethyl) phosphine hydrochloride, (TCEP, CAS 5961-85-3), cysteine hydrochloride (Cys-HCl, CAS 52-89-1), or 2-mercaptoethanesulfonic acid sodium salt (MESNA). Other methods for reducing protein bonds are known in the art, such as an immobilized reductant column which contains resin to which a thiol-based reducing agent has been immobilized to enable the solid-phase reduction of peptide and protein disulfide bonds. Additional reducing agents suitable for cleaving disulfide bonds are also envisioned.

According to the invention, the reducing buffer contains dithiothreitol. In an embodiment, the reducing buffer contains 80 to 155 mM dithiothreitol, with the proviso that the buffer does not contain 135 to 155 mM dithiothreitol.

Still another embodiment provides a reducing buffer containing 1.2 % lithium dodecyl sulfate, 60 mM sodium phosphate, and 80 mM dithiothreitol.

### 3. HEPES Based Non-Reducing Buffers

HEPES based buffers are not part of the claimed subject-matter but can also be used with the disclosed methods. One embodiment provides a non-reducing HEPES based aqueous electrophoresis sample buffer containing an alkylating agent, for example 55 to 75 mM 2-iodoacetamide; 0.1 to 1.0 % lithium dodecyl sulfate; 5 to 85 mM HEPES, and 5 to 115 mM sodium chloride, wherein the aqueous electrophoresis sample buffer has a pH of less than 9. In an embodiment, the pH of the buffer is 8. In another embodiment, the aqueous buffer contains 66.4 mM 2-iodoacetamide, 0.32% lithium dodecyl sulfate, 16.2 mM HEPES, and 48.6 mM sodium chloride.

### 4. HEPES Based Reducing Buffers

Another embodiment provides a reducing HEPES based aqueous electrophoresis sample buffer containing 0.05 to 0.75 % lithium dodecyl sulfate, 5 mM to 115 mM sodium chloride, 5 mM to 115 mM HEPES, and a reducing agent, wherein the aqueous electrophoresis sample buffer has a pH greater than 7. In an embodiment, the pH of the buffer is 8. In one embodiment, the reducing buffer contains 35 to 50 mM dithiothreitol. Still another embodiment provides a reducing buffer containing 0.28 % lithium dodecyl sulfate, 41.5 mM sodium chloride, 13.8 mM HEPES, and 42.5 mM dithiothreitol.

### B. Assays

### 1. Non-Reducing Assays

One embodiment provides a non-reducing MCE method for identifying contaminants or impurities in a protein drug sample, the method including the steps of adding the protein sample to a non-reducing buffer discussed above to form a buffered protein drug sample. The buffered protein drug sample is heated to between 50 to 85 °C for 5 to 15 minutes to form a denatured buffered protein drug sample, with the proviso that the buffered protein drug sample is not heated to between 65 to 85 °C for 5 to 15 minutes to form the denatured buffered protein drug sample. In another embodiment, the buffered protein drug sample is heated to between 45 to 75 °C for 5 to 15 minutes to form a denatured buffered protein drug sample, with the proviso that the buffered protein drug sample is not heated to between 65 to 85 °C for 5 to 15 minutes to form the denatured buffered protein drug sample. In another preferred embodiment, the buffered protein drug sample is heated to 60 °C for 10 min. In yet another preferred embodiment, the buffered protein drug sample is heated to 63 °C for 10 min.

A detectable label is prepared to vendor recommendations in dimethyl sulfoxide (DMSO). Subsequent dilutions are prepared per vendor recommendations in MilliQ. In other embodiments, the label is prepared by dilution in 200 mM sodium phosphate pH 7.2. In some embodiments, the label is diluted to a 5 µM solution. In other embodiments, the label is diluted to a 16 µM solution. The diluted label is then added to the denatured buffered protein drug sample and heated at 30 to 40 °C for 10 to 40 minutes to form a denatured labeled protein drug sample. In another embodiment, a detectable label is then added to the denatured buffered protein drug sample and heated at 30 to 40 °C for 15 minutes to form a denatured labeled protein drug sample. In an embodiment, the denatured protein drug sample with added label is heated to 35 °C for 30 minutes. In another preferred embodiment, the denatured protein drug sample with added label is heated to 35 °C for 15 minutes. Excess label is optionally removed from the sample, for example by using a spin filter. In still another embodiment, excess label is not quenched. In still another embodiment, excess label is not removed.

A preferred detectable label includes, but is not limited to Dyomics DY-631 NHS Ester. Other detectable labels can be used include other dyes, fluorophores, chromophores, mass tags, quantum dots and the like and those disclosed in US Patent No. 6,924,372.

The denatured labeled protein drug product is diluted and subjected to MCE to separate the diluted protein drug sample on a microchip capillary electrophoresis system to produce an electropherogram. In one embodiment the final concentration of a sample starting at 0.5 mg/ml that is then injected over the microchip is 9 µg/ml to MCE. In another embodiment, the sample starting concentration is 0.2 mg/ml. The electropherogram contains peaks corresponding to the protein drug product and impurities. The method concludes by identifying peaks in the electropherogram corresponding to contaminants or impurities.

### 2. Reducing Assays

Another embodiment provides a reducing MCE method for identifying contaminants or impurities in a protein drug sample. The method begins by adding the protein drug sample to any one of the reducing buffers described above to form a buffered protein drug sample. In an embodiment, the buffered protein drug sample is denatured by heating the buffered protein drug sample to 50 to 72 °C, or 45 to 75 °C, or preferably to 60 °C for 10 minutes to form a denatured protein drug sample, with the proviso that the buffered protein drug sample is not heated to between 65 to 85 °C for 5 to 15 minutes to form the denatured buffered protein drug sample. In yet another preferred embodiment, the buffered protein drug sample is heated to 63 °C for 10 min. The temperature needed to denature the target protein drug sample may be changed according to the structure of the target protein drug sample.

The protein drug sample with added label is then heated at 30 to 40 °C for 20 to 40 minutes or for 10 to 20 or for 10 to 40 minutes to form a denatured labeled protein drug sample. In an embodiment, the protein drug product sample with added label is heated to 35 °C for 30 minutes. In another preferred embodiment, the protein drug product sample with added label is heated to 35 °C for 15 minutes. Excess label is optionally removed from the sample, for example by using a spin filter. In still another embodiment, excess label is not quenched. In still another embodiment, excess label is not removed. A preferred detectable label includes, but is not limited to Dyomics DY-631 NHS Ester. Other detectable labels that can be used include other dyes, fluorophores, chromophores, mass tags, quantum dots and the like and those disclosed in US Patent No. 6,924,372.

In one embodiment, the established assay range for sample concentration is from 0.4 mg/ml to 0.6 mg/ml, corresponding to a final concentration being analyzed of about 7µg/ml to 11µg/ml which is subjected to MCE analysis on a microchip capillary electrophoresis system to produce an electropherogram. In another embodiment, the established assay range for sample concentration is from 0.2 mg/ml to 0.6 mg/ml, corresponding to a final concentration being analyzed of about 3.6 µg/ml to 11 µg/ml which is subjected to MCE analysis on a microchip capillary electrophoresis system to produce an electropherogram. The method concludes by identifying peaks in the electropherogram corresponding to protein drug product, contaminants, and/or impurities.

### C. Instrumentation

Instrumentation for conducting the disclosed MCE assays is commercially available. In an embodiment, the disclosed MCE assays are performed using LabChip GXII or LabChip GXII Touch HT and LabChip^{®} HT Protein Express Chip.

### III. Proteins of Interest

The protein of interest, for example a protein drug product, assayed with the disclosed MCE assays and reagents can be any protein of interest suitable for expression in prokaryotic or eukaryotic cells and can be used in the engineered host cell systems provided. For example, the protein of interest includes, but is not limited to, an antibody or antigen-binding fragment thereof, a chimeric antibody or antigen-binding fragment thereof, an ScFv or fragment thereof, an Fc-fusion protein or fragment thereof, a growth factor or a fragment thereof, a cytokine or a fragment thereof, or an extracellular domain of a cell surface receptor or a fragment thereof. Proteins of interest may be simple polypeptides consisting of a single subunit, or complex multisubunit proteins comprising two or more subunits. The protein of interest may be a biopharmaceutical product, food additive or preservative, or any protein product subject to purification and quality standards.

In some embodiments, the protein drug product (protein of interest) is an antibody, a human antibody, a humanized antibody, a chimeric antibody, a monoclonal antibody, a multispecific antibody, a bispecific antibody, an antigen binding antibody fragment, a single chain antibody, a diabody, triabody or tetrabody, a Fab fragment or a F(ab')2 fragment, an IgD antibody, an IgE antibody, an IgM antibody, an IgG antibody, an IgG1 antibody, an IgG2 antibody, an IgG3 antibody, or an IgG4 antibody. In one embodiment, the antibody is an IgG1 antibody. In one embodiment, the antibody is an IgG2 antibody. In one embodiment, the antibody is an IgG4 antibody. In one embodiment, the antibody is a chimeric IgG2/IgG4 antibody. In one embodiment, the antibody is a chimeric IgG2/IgG1 antibody. In one embodiment, the antibody is a chimeric IgG2/IgG1/IgG4 antibody.

Embodiments can be used with any known therapeutic antibody therapeutic. In some embodiments, the antibody can be selected from the group consisting of an anti-Programmed Cell Death 1 antibody (e.g. an anti-PD1 antibody as described in U.S. Pat. Appln. Pub. No. US2015/0203579A1), an anti-Programmed Cell Death Ligand-1 (e.g., an anti-PD-L1 antibody as described in in U.S. Pat. Appln. Pub. No. US2015/0203580A1), an anti-Dll4 antibody, an anti-Angiopoetin-2 antibody (e.g., an anti-ANG2 antibody as described in U.S. Pat. No. 9,402,898), an anti- Angiopoetin-Like 3 antibody (e.g., an anti-AngPtl3 antibody as described in U.S. Pat. No. 9,018,356), an anti-platelet derived growth factor receptor antibody (e.g., an anti-PDGFR antibody as described in U.S. Pat. No. 9,265,827), an anti-Erb3 antibody, an anti- Prolactin Receptor antibody (e.g., anti-PRLR antibody as described in U.S. Pat. No. 9,302,015), an anti-Complement 5 antibody (e.g., an anti-C5 antibody as described in U.S. Pat. Appln. Pub. No US2015/0313194A1), an anti-TNF antibody, an anti-epidermal growth factor receptor antibody (e.g., an anti-EGFR antibody as described in U.S. Pat. No. 9,132,192 or an anti-EGFRvIII antibody as described in U.S. Pat. Appln. Pub. No. US2015/0259423A1), an anti-Proprotein Convertase Subtilisin Kexin-9 antibody (e.g., an anti-PCSK9 antibody as described in U.S. Pat. No. 8,062,640 or U.S. Pat. No. 9,540,449), an Anti-Growth and Differentiation Factor-8 antibody (e.g. an anti-GDF8 antibody, also known as anti-myostatin antibody, as described in U.S. Pat Nos. 8,871,209 or 9,260,515), an anti-Glucagon Receptor (e.g. anti-GCGR antibody as described in U.S. Pat. Appln. Pub. Nos. US2015/0337045A1 or US2016/0075778A1), an anti-VEGF antibody, an anti-IL1R antibody, an interleukin 4 receptor antibody (e.g., an anti-IL4R antibody as described in U.S. Pat. Appln. Pub. No. US2014/0271681A1 or U.S. Pat Nos. 8,735,095 or 8,945,559), an anti-interleukin 6 receptor antibody (e.g., an anti-IL6R antibody as described in U.S. Pat. Nos. 7,582,298, 8,043,617 or 9,173,880), an anti-IL1 antibody, an anti-IL2 antibody, an anti-IL3 antibody, an anti-IL4 antibody, an anti-IL5 antibody, an anti-IL6 antibody, an anti-IL7 antibody, an anti-interleukin 33 (e.g., anti- IL33 antibody as described in U.S. Pat. Nos. 9,453,072 or 9,637,535), an anti-Respiratory syncytial virus antibody (e.g., anti-RSV antibody as described in U.S. Pat. Appln. Pub. No. 9,447,173), an anti-Cluster of differentiation 3 (e.g., an anti-CD3 antibody, as described in U.S. Pat. Nos. 9,447,173and 9,447,173, and in U.S. Application No. 62/222,605), an anti- Cluster of differentiation 20 (e.g., an anti-CD20 antibody as described in U.S. Pat. Nos. 9,657,102 and US20150266966A1, and in U.S. Pat. No. 7,879,984), an anti-CD19 antibody, an anti-CD28 antibody, an anti- Cluster of Differentiation-48 (e.g. anti-CD48 antibody as described in U.S. Pat. No. 9,228,014), an anti-Fel d1 antibody (e.g. as described in U.S. Pat. No. 9,079,948), an anti-Middle East Respiratory Syndrome virus (e.g. an anti-MERS antibody as described in U.S. Pat. Appln. Pub. No. US2015/0337029A1), an anti-Ebola virus antibody (e.g. as described in U.S. Pat. Appln. Pub. No. US2016/0215040), an anti-Zika virus antibody, an anti-Lymphocyte Activation Gene 3 antibody (e.g. an anti-LAG3 antibody, or an anti-CD223 antibody), an anti-Nerve Growth Factor antibody (e.g. an anti-NGF antibody as described in U.S. Pat. Appln. Pub. No. US2016/0017029 and U.S. Pat. Nos. 8,309,088 and 9,353,176) and an anti-Protein Y antibody. In some embodiments, the bispecific antibody can be selected from the group consisting of an anti-CD3 x anti-CD20 bispecific antibody (as described in U.S. Pat. Appln. Pub. Nos. US2014/0088295A1 and US20150266966A1), an anti-CD3 x anti-Mucin 16 bispecific antibody (e.g., an anti-CD3 x anti-Muc16 bispecific antibody), and an anti-CD3 x anti- Prostate-specific membrane antigen bispecific antibody (e.g., an anti-CD3 x anti-PSMA bispecific antibody). In some embodiments, the protein of interest can be selected from the group consisting of casirivimab, imdevimab, ravulizumab-cwvz, abciximab, adalimumab, adalimumab-atto, ado-trastuzumab, alemtuzumab, alirocumab, atezolizumab, avelumab, basiliximab, belimumab, benralizumab, bevacizumab, bezlotoxumab, blinatumomab, brentuximab vedotin, brodalumab, canakinumab, capromab pendetide, certolizumab pegol, cemiplimab, cetuximab, denosumab, dinutuximab, dupilumab, durvalumab, eculizumab, elotuzumab, emicizumab-kxwh, emtansinealirocumab, evinacumab, evolocumab, fasinumab, golimumab, guselkumab, ibritumomab tiuxetan, idarucizumab, infliximab, infliximab-abda, infliximab-dyyb, ipilimumab, ixekizumab, mepolizumab, necitumumab, nesvacumab, nivolumab, obiltoxaximab, obinutuzumab, ocrelizumab, ofatumumab, olaratumab, omalizumab, panitumumab, pembrolizumab, pertuzumab, ramucirumab, ranibizumab, raxibacumab, reslizumab, rinucumab, rituximab, sarilumab, secukinumab, siltuximab, tocilizumab, tocilizumab, trastuzumab, trevogrumab, ustekinumab, and vedolizumab.

In some embodiments, the protein of interest is a recombinant protein that contains an Fc moiety and another domain, (e.g., an Fc-fusion protein). In some embodiments, an Fc-fusion protein is a receptor Fc-fusion protein, which contains one or more extracellular domain(s) of a receptor coupled to an Fc moiety. In some embodiments, the Fc moiety comprises a hinge region followed by a CH2 and CH3 domain of an IgG. In some embodiments, the receptor Fc-fusion protein contains two or more distinct receptor chains that bind to either a single ligand or multiple ligands. For example, an Fc-fusion protein is a TRAP protein, such as an IL-1 trap (e.g., rilonacept, which contains the IL-1RAcP ligand binding region fused to the Il-1R1 extracellular region fused to Fc of hIgG1; see U.S. Pat. No. 6,927,044, or a VEGF trap (e.g., aflibercept or ziv-aflibercept, which comprises the Ig domain 2 of the VEGF receptor Flt1 fused to the Ig domain 3 of the VEGF receptor Flk1 fused to Fc of hIgG1; see U.S. Pat. Nos. 7,087,411 and 7,279,159). In other embodiments, an Fc-fusion protein is a ScFv-Fc-fusion protein, which contains one or more of one or more antigen-binding domain(s), such as a variable heavy chain fragment and a variable light chain fragment, of an antibody coupled to an Fc moiety.

### IV. Cell Culture

The protein drug product assayed with the disclosed MCE assays and reagents are produced cell cultures. The cell cultures can be a "fed-batch cell culture" or "fed-batch culture" which refers to a batch culture wherein the cells and culture medium are supplied to the culturing vessel initially and additional culture nutrients are slowly fed, in discrete increments, to the culture during culturing, with or without periodic cell and/or product harvest before termination of culture. Fed-batch culture includes "semi-continuous fed-batch culture" wherein periodically whole culture (which may include cells and medium) is removed and replaced by fresh medium. Fed-batch culture is distinguished from simple "batch culture" whereas all components for cell culturing (including the animal cells and all culture nutrients) are supplied to the culturing vessel at the start of the culturing process in batch culture. Fed-batch culture may be different from "perfusion culture" insofar as the supernatant is not removed from the culturing vessel during a standard fed-batch process, whereas in perfusion culturing, the cells are restrained in the culture by, e.g., filtration, and the culture medium is continuously or intermittently introduced and removed from the culturing vessel. However, removal of samples for testing purposes during fed-batch cell culture is contemplated. The fed-batch process continues until it is determined that maximum working volume and/or protein production is reached, and protein is subsequently harvested.

The cell culture can be a "continuous cell culture" which is a technique used to grow cells continually, usually in a particular growth phase. For example, if a constant supply of cells is required, or the production of a particular protein of interest is required, the cell culture may require maintenance in a particular phase of growth. Thus, the conditions must be continually monitored and adjusted accordingly in order to maintain the cells in that particular phase.

The cells are cultured in cell culture medium. The terms "cell culture medium" and "culture medium" refer to a nutrient solution used for growing mammalian cells that typically provides the necessary nutrients to enhance growth of the cells, such as a carbohydrate energy source, essential (e.g., phenylalanine, valine, threonine, tryptophan, methionine, leucine, isoleucine, lysine, and histidine) and nonessential (e.g., alanine, asparagine, aspartic acid, cysteine, glutamic acid, glutamine, glycine, proline, serine, and tyrosine) amino acids, trace elements, energy sources, lipids, vitamins, etc. Cell culture medium may contain extracts, e.g., serum or peptones (hydrolysates), which supply raw materials that support cell growth. Media may contain yeast-derived or soy extracts, instead of animal-derived extracts. Chemically defined medium refers to a cell culture medium in which all of the chemical components are known (i.e., have a known chemical structure). Chemically defined medium is entirely free of animal-derived components, such as serum- or animal-derived peptones. In one embodiment, the medium is a chemically defined medium.

The solution may also contain components that enhance growth and/or survival above the minimal rate, including hormones and growth factors. The solution may be formulated to a pH and salt concentration optimal for survival and proliferation of the particular cell being cultured.

A "cell line" refers to a cell or cells that are derived from a particular lineage through serial passaging or subculturing of cells. The term "cells" is used interchangeably with "cell population".

The term "cell" includes any cell that is suitable for expressing a recombinant nucleic acid sequence. Cells include those of prokaryotes and eukaryotes, such as bacterial cells, mammalian cells, human cells, non-human animal cells, avian cells, insect cells, yeast cells, or cell fusions such as, for example, hybridomas or quadromas. In certain embodiments, the cell is a human, monkey, ape, hamster, rat or mouse cell. In other embodiments, the cell can be selected from the following cells: Chinese Hamster Ovary (CHO) (e.g., CHO K1, DXB-11 CHO, Veggie-CHO), COS (e.g., COS-7), retinal cell, Vero, CV1, kidney (e.g., HEK293, 293 EBNA, MSR 293, MDCK, HaK, BHK21), HeLa, HepG2, WI38, MRC 5, Colo25, HB 8065, HL-60, lymphocyte, e.g., Jurkat (T lymphocyte) or Daudi (B lymphocyte), A431 (epidermal), U937, 3T3, L cell, C127 cell, SP2/0, NS-0, MMT cell, stem cell, tumor cell, and a cell line derived from an aforementioned cell. In some embodiments, the cell comprises one or more viral genes, e.g., a retinal cell that expresses a viral gene (e.g., a PER.C6^{®} cell). In some embodiments, the cell is a CHO cell. In other embodiments, the cell is a CHO K1 cell.

### V. Kits

One embodiment provides a kit including the one or more of the disclosed buffers or ingredients to make the disclosed buffers. The kit can include a container for the buffers or ingredients. The buffers can be in solution or in lyophilized form. The kit optionally also includes a second container containing a diluent or reconstituting solution for the lyophilized formulation; and optionally, instructions for the use of the solution or the reconstitution and/or use of the lyophilized buffers or powdered ingredients.

The kit may further include additional reagents needed to perform the disclosed MCE assays including one or more of a buffer, a diluent, and a filter. The buffer and reagents can be in a bottle, a vial, or test tube.

The following examples are not intended to limit the scope of what the inventors regard as their inventions.

### EXAMPLES

### Example 1: MCE assay for purity and impurity analysis of therapeutic proteins. Methods and Materials:

### Materials:

LabChip GXII or LabChip GXII Touch HT and LabChip^{®} HT Protein Express Chip were used for capillary electrophoretic separation and data collection (Perkin Elmer). Non-reducing and reducing denaturing buffers disclosed above were used for the MCE assay.

### Methods:

Microchip Capillary Electrophoresis (MCE) provides analysis of protein purity and impurities suitable for in-process testing, lot release, and stability indication. Protein samples of casirivimab and imdevimab were prepared by denaturing the sample through heating and addition of lithium dodecyl sulfate (LDS) with either an alkylating (non-reduced) or reducing agent (reduced). The resulting samples were then incubated with an amine ester dye to covalently label any free amine groups. Labeled samples were injected onto a microchip where the application of a current separated the sample components according to size (from low to high molecular weight). Detection was performed by laser-induced fluorescence, producing an electropherogram. Analysis of the electropherogram provided the relative purity of the sample compared to the impurities present.

Table 1 shows the workflow procedure for preparing a sample for a first MCE assay. Briefly, protein samples were diluted to 0.5 mg/ml. 1 µl of either non-reducing (NR) or reducing (R) denaturing buffer and 4 µl of the diluted sample were added to a 96-well plate. The sample was mixed, centrifuged, and heated for 10 minutes at the temperature specified for the product, typically 75°C. The samples were then labeled with 5 µM commercially available dye (for example Dyomics DY-631 NHS Ester, also called PICO dye). The samples were mixed, centrifuged, and then heated at 35°C for 30 minutes. The labeled sample was then diluted with 105 µl of dilute stop solution. The samples were separated using LabChip GXII or LabChip GXII Touch HT.

**Table 1. Sample preparation method for a first MCE assay.**

| **Sample Preparation** | |
|---|---|
| NR | R |
| 4 µl 0.5 mg/mL Sample | 4 µl 0.5 mg/mL Sample |
| 1 µL NR Buffer | 1 µL R Buffer |
| Mix, centrifuge, heat at Specified Temperature for 10 minutes | |

| **Sample Labeling** | |
|---|---|
| 5 µL Denatured Sample | |
| 5 µL 5 µM PICO dye | |
| Mix, centrifuge, heat at 35°C for 30 minutes | |

| **Final Dilution** | |
|---|---|
| 5 µL Labeled Sample | |
| 105 µL Dilute Stop Solution | |
| **Separation Method** | |
| HT PICO Protein Express 200 | |

Table 2 shows the workflow procedure for preparing a sample for a second MCE assay. Briefly, protein samples were diluted to 0.5 or 2 mg/ml. 10 µl of either non-reducing (NR) or reducing (R) denaturing buffer and 40 µl of the diluted sample were added to a 96-well plate. The sample was mixed, centrifuged, and heated for 10 minutes at the temperature specified for the product, typically 60°C. The samples were then labeled with 16 µM commercially available dye (for example Dyomics DY-631 NHS Ester). The samples were mixed, centrifuged, and then heated at 35°C for 15 minutes. The labeled sample was then diluted with 105 µl of dilute stop solution or dilute solution. The samples were separated using LabChip GXII or LabChip GXII Touch HT.

### Example 1A: Non-reducing buffer

The general preparation presented in Table 1 was followed, using in the Sample Preparation Step a non-reducing aqueous electrophoresis sample buffer that contained 166 mM 2-iodoacetamide, 0.81% lithium dodecyl sulfate, and 81 mM sodium phosphate and had a pH of 6. The sample was heated at 75_°C for 10 minutes. The Sample Labeling step used 5 µM dye solution. The Final Dilution step used 105 µL Dilute Stop Solution.

### Example 1B: Reducing buffer

The general preparation presented in Table 1 was followed, using in the Sample Preparation Step a reducing aqueous electrophoresis sample buffer that contained 0.69% lithium dodecyl sulfate, 69 mM sodium phosphate, and 142 mM dithiothreitol, and had a pH of 9. The sample was heated at 75°C for 10 minutes. The Sample Labeling step used 5 µM dye solution. The Final Dilution step used 105 µL Dilute Stop Solution.

**Table 2. General sample preparation method for a second MCE assay.**

| **Sample Preparation** | |
|---|---|
| NR | R |
| 40 µl 0.2 mg/mL Sample | 40 µl 0.2 mg/mL Sample |
| 10 µL NR Buffer | 10 µL R Buffer |
| Mix, centrifuge, heat at Specified Temperature for 10 minutes | |

| **Sample Labeling** | |
|---|---|
| 5 µL Denatured Sample | |
| 16 µL 16 µM PICO dye | |
| Mix, centrifuge, heat at 35°C for 15 minutes | |

| **Final Dilution** | |
|---|---|
| 5 µL Labeled Sample | |
| 105 µL Dilute Stop Solution or Dilute Solution | |

| **Separation Method** | |
|---|---|
| HT PICO Protein Express 200 | |

### Buffers

Stock solutions of 200 mM Sodium Phosphate Monobasic Monohydrate, 200 mM Sodium Phosphate Dibasic Hepathydrate, and 10% Lithium Dodecyl Sulfate (LDS) were prepared. Alternately, a commercially prepared sodium phosphate buffer with a pH of 8 was used.

Using the stock solutions and Milli-Q^{®} water, solutions of 100 mM Sodium Phosphate 1% LDS pH 6 and 100 mM Sodium Phosphate 1 % LDS pH 9 were prepared.

Alternate buffers were made using stock solutions and Milli-Q water include solutions of 100 mM Sodium Phosphate 2 % LDS pH 6 and 100 mM Sodium Phosphate 2% LDS pH 8.

A non-reducing buffer was prepared by adding 34 µL 1M Iodoacetamide (IAM) (prepared fresh in Milli-Q^{®} water) + 166 µL 100 mM Sodium Phosphate 1% LDS pH 6 + 5 µL Milli-Q^{®} water. The final concentrations were 166 mM 2-iodoacetamide, 0.81 % lithium dodecyl sulfate, and 81 mM Sodium phosphate.

An alternate non-reducing buffer was prepared by adding 800 µL 1M Iodoacetamide (IAM) (prepared fresh in Milli-Q^{®} water) + 1200 µL 100 mM Sodium Phosphate 2% LDS pH 8. The final concentrations were 200 mM 2-iodoacetamide, 1.2 % lithium dodecyl sulfate, and 60 mM Sodium phosphate.

Yet another non-reducing buffer was prepared by adding 800 µL 1M Iodoacetamide (IAM) (prepared fresh in Milli-Q^{®} water) + 1200 µL 100 mM Sodium Phosphate 2% LDS pH 6. The final concentrations were 200 mM 2-iodoacetamide, 1.2 % lithium dodecyl sulfate, and 60 mM Sodium phosphate.

A reducing buffer was prepared by adding 68 µL 10x Reducing agent (500 mM dithiothreitol (DTT) + 166 µL 100 mM Sodium Phosphate 1% LDS pH 9 + 6 µL Milli-Q^{®} water. The final concentrations were 0.69% lithium dodecyl sulfate; 69 mM sodium phosphate, and 142 mM dithiothreitol.

An alternate reducing buffer was prepared by adding 320 µL 10x Reducing agent (500 mM dithiothreitol (DTT) + 1200 µL 100 mM Sodium Phosphate 2% LDS pH 8 + 480 µL MilliQ^{®} water. The final concentrations were 1.2% lithium dodecyl sulfate; 60 mM sodium phosphate, and 80 mM dithiothreitol.

### Example 2A: Non-reducing buffer

The general preparation presented in Table 2 was followed, using in the Sample Preparation Step a non-reducing aqueous electrophoresis sample buffer that contained 200 mM 2-iodoacetamide, 1.2% lithium dodecyl sulfate, and 60 mM sodium phosphate and had a pH of 8. The sample was heated at 60°C for 10 minutes. The Sample Labeling step used 16 µM dye solution. The Final Dilution step used 105 µL Dilute Stop Solution.

### Example 2B: Reducing buffer

The general preparation presented in Table 2 was followed, using in the Sample Preparation Step a reducing aqueous electrophoresis sample buffer that contained 1.2% lithium dodecyl sulfate, 60 mM sodium phosphate, and 80 mM dithiothreitol, and had a pH of 8. The sample was heated at 60°C for 10 minutes. The Sample Labeling step used 16 µM dye solution. The Final Dilution step used 105 µL Dilute Stop Solution.

### Example 2C: Non-reducing buffer, non-quenching

The general preparation presented in Table 2 was followed, using in the Sample Preparation Step a non-reducing aqueous electrophoresis sample buffer that contained 200 mM 2-iodoacetamide, 1.2% lithium dodecyl sulfate, and 60 mM sodium phosphate and had a pH of 6. The sample was heated at 63°C for 10 minutes. The Sample Labeling step used 16 µM dye solution. The Final Dilution step used 105 µL Dilute Solution.

### Example 2D: Reducing buffer, non-quenching

The general preparation presented in Table 2 was followed, using in the Sample Preparation Step a reducing aqueous electrophoresis sample buffer that contained 1.2% lithium dodecyl sulfate, 60 mM sodium phosphate, and 80 mM dithiothreitol, and had a pH of 8. The sample was heated at 63°C for 10 minutes. The Sample Labeling step used 16 µM dye solution. The Final Dilution step used 105 µL Dilute Solution.

### Results:

Microchip Capillary Electrophoresis (MCE) allows for dramatically reduced sample analysis times, while maintaining the performance and reproducibility standards required for QC analysis. An MCE assay was developed using the non-reduced and reduced denaturing buffers disclosed herein. Figures 1A-1B show representative electropherograms of protein in non-reduced samples and reduced samples from the first MCE assay. Figure 1A shows a non-reduced analysis electropherogram. The main peak (MP) at 0.426 corresponds to the main antibody, and the labeled low molecular weight (LMW) peaks corresponds to antibody fragments. Although not present in this example, in some instances a peak corresponding to one or more high molecular weight fragments are present. Figure IB shows a reduced analysis electropherogram. Peaks corresponding to light chain (LC), heavy chain, and non-glycosylated heavy chain (NGHC), low molecular weight (LMW), and high molecular weight (HMW) antibody fragments are labeled accordingly. It should be noted that different products may have variation in the electropherograms produced, and therefore different labeling conventions.

The present invention is defined in the appended claims.

## Claims

1. An aqueous electrophoresis sample buffer, comprising:
155 to 200 mM 2-iodoacetamide;
0.50 to 1.5% lithium dodecyl sulfate; and
60 to 95 mM sodium phosphate,
wherein the aqueous electrophoresis sample buffer has a pH of less than or equal to 8, with the proviso that it is not an aqueous electrophoresis sample buffer with a pH of less than 7, and comprising:
155 to 175 mM 2-iodoacetamide;
0.50 to 1.5% lithium dodecyl sulfate; and
and 65 to 95 mM sodium phosphate.

2. The aqueous buffer of claim 1, wherein the pH is 6.

3. The aqueous buffer of claim 1, comprising 200 mM 2-iodoacetamide, 1.2% lithium dodecyl sulfate, and 60 mM sodium phosphate.

4. The aqueous electrophoresis sample buffer of claim 1, comprising:
200 mM 2-iodoacetamide,
1.2 % lithium dodecyl sulfate, and
60 mM Sodium phosphate,
wherein the aqueous electrophoresis sample buffer has a pH of 6.0; or comprising:
200 mM 2-iodoacetamide,
1.2 % lithium dodecyl sulfate, and
60 mM Sodium phosphate,
wherein the aqueous electrophoresis sample buffer has a pH of 8.0.

5. An aqueous electrophoresis sample buffer, comprising:
0.50 to 1.5% lithium dodecyl sulfate;
45 to 75 mM sodium phosphate,
80 to 155 mM dithiothreitol,
wherein the aqueous electrophoresis sample buffer has a pH of 8 or greater, for example wherein the pH is 8 or 9, and
with the proviso that the aqueous electrophoresis sample buffer does not comprise 135 to 155 mM dithiothreitol.

6. The aqueous buffer of claim 5, comprising 80 mM dithiothreitol.

7. The aqueous buffer of claim 5, further comprising 1.2 % lithium dodecyl sulfate and 60 mM sodium phosphate.

8. The aqueous buffer of claim 5, consisting of:
0.69% lithium dodecyl sulfate;
69 mM sodium phosphate, and
80 to 155 mM dithiothreitol,
wherein the aqueous electrophoresis sample buffer has a pH of 9.0; or consisting of: 1.2% lithium dodecyl sulfate;
60 mM sodium phosphate, and
80 mM dithiothreitol,
wherein the aqueous electrophoresis sample buffer has a pH of 8.0.

9. A method for identifying contaminants or impurities in a protein drug sample, the method comprising the steps of:
adding the protein drug sample to the buffer of any of claims 1 to 8 to form a buffered protein drug sample;
heating the buffered protein drug sample to 45 to 75°C for 5 to 15 minutes to form a denatured buffered protein drug sample;
adding a detectable label to the denatured buffered protein drug sample and heating it at 30 to 40 °C for 10 to 40 minutes to form a denatured labeled protein drug sample;
diluting the denatured labeled protein drug sample and subjecting it to either (a) microchip capillary electrophoresis (MCE) to separate the diluted protein drug sample on a microchip capillary electrophoresis system to produce an electropherogram or (b) MCE analysis on a microchip capillary electrophoresis system to produce an electropherogram; and
identifying peaks in the electropherogram corresponding to the protein drug, contaminants, and/or impurities,
with the proviso that the buffered protein drug sample is not heated to between 65 to 85 °C for 5 to 15 minutes to form the denatured buffered protein drug sample.

10. The method of claim 9, wherein the buffered protein drug sample is heated at 60 °C for 10 minutes and/or wherein the labeled protein drug sample is heated at 35 °C for 15 minutes.

11. The method of claim 9, wherein the diluted protein drug sample has a concentration of 3.6 µg/ml.

12. The method of any of claims 9 to 11, wherein the detectable label is DY-631 N-hydroxysuccinimidyl ester.

13. The method of any of claims 9 to 11, wherein the detectable label is reconstituted with dimethyl sulfoxide (DMSO) followed by dilution with 200 mM Sodium Phosphate pH 7.2 buffer.

14. A kit comprising the buffer according to any of claims 1 to 8, and
written instructions for preparing a sample for electrophoresis in the buffer.

## Patentansprüche

1. Wässriger Elektrophoreseprobenpuffer, umfassend:
155 bis 200 mM 2-Iodoacetamid;
0,50 bis 1,5 % Lithiumdodecylsulfat; und
60 bis 95 mM Natriumphosphat,
wobei der wässrige Elektrophoreseprobenpuffer einen pH-Wert von weniger als oder gleich 8 aufweist,
unter der Bedingung, dass es sich nicht um einen wässrigen Elektrophoreseprobenpuffer mit einem pH-Wert von weniger als 7 handelt, und umfassend:
155 bis 175 mM 2-Iodoacetamid;
0,50 bis 1,5 % Lithiumdodecylsulfat; und
und 65 bis 95 mM Natriumphosphat.

2. Wässriger Puffer nach Anspruch 1, wobei der pH-Wert 6 beträgt.

3. Wässriger Puffer nach Anspruch 1, umfassend 200 mM 2-lodoacetamid, 1,2 % Lithiumdodecylsulfat und 60 mM Natriumphosphat.

4. Wässriger Elektrophoreseprobenpuffer nach Anspruch 1, umfassend:
200 mM 2-Iodoacetamid,
1,2 % Lithiumdodecylsulfat und
60 mM Natriumphosphat,
wobei der wässrige Elektrophoreseprobenpuffer einen pH-Wert von 6,0 aufweist; oder umfassend:
200 mM 2-Iodoacetamid,
1,2 % Lithiumdodecylsulfat und
60 mM Natriumphosphat,
wobei der wässrige Elektrophoreseprobenpuffer einen pH-Wert von 8,0 aufweist.

5. Wässriger Elektrophoreseprobenpuffer, umfassend:
0,50 bis 1,5 % Lithiumdodecylsulfat;
45 bis 75 mM Natriumphosphat,
80 bis 155 mM Dithiothreitol,
wobei der wässrige Elektrophoreseprobenpuffer einen pH-Wert von 8 oder höher aufweist, beispielsweise wobei der pH-Wert 8 oder 9 ist, und
mit der Bedingung, dass der wässrige Elektrophoreseprobenpuffer nicht 135 bis 155 mM Dithiothreitol umfasst.

6. Wässriger Puffer nach Anspruch 5, umfassend 80 mM Dithiothreitol.

7. Wässriger Puffer nach Anspruch 5, weiter umfassend 1,2 % Lithiumdodecylsulfat und 60 mM Natriumphosphat.

8. Wässriger Puffer nach Anspruch 5, bestehend aus:
0,69 % Lithiumdodecylsulfat;
69 mM Natriumphosphat und
80 bis 155 mM Dithiothreitol,
wobei der wässrige Elektrophoreseprobenpuffer einen pH-Wert von 9,0 aufweist; oder bestehend aus:
1,2 % Lithiumdodecylsulfat;
60 mM Natriumphosphat, und
80 mM Dithiothreitol,
wobei der wässrige Elektrophoreseprobenpuffer einen pH-Wert von 8,0 aufweist.

9. Verfahren zum Identifizieren von Verunreinigungen oder Unreinheiten in einer Proteinarzneimittelprobe, wobei das Verfahren folgende Schritte umfasst:
Hinzufügen der Proteinarzneimittelprobe zum Puffer nach einem der Ansprüche 1 bis 8, um eine gepufferte Proteinarzneimittelprobe zu bilden;
Erhitzen der gepufferten Proteinarzneimittelprobe für 5 bis 15 Minuten auf 45 bis 75 °C, um eine denaturierte gepufferte Proteinarzneimittelprobe zu bilden;
Hinzufügen eines nachweisbaren Etiketts zu der denaturierten gepufferten Proteinarzneimittelprobe und Erhitzen für 10 bis 40 Minuten bei 30 bis 40 °C, um eine denaturierte etikettierte Proteinarzneimittelprobe zu bilden;
Verdünnen der denaturierten etikettierten Proteinarzneimittelprobe und Unterziehen derselben entweder (a) einer Mikro-Chip-Kapillarelektrophorese (MCE), um die verdünnte Proteinarzneimittelprobe auf einem Mikro-Chip-Kapillarelektrophoresesystem zu trennen, um ein Elektropherogramm herzustellen, oder (b) einer MCE-Analyse auf einem Mikro-Chip-Kapillarelektrophoresesystem, um ein Elektropherogramm herzustellen; und Identifizieren von Peaks in dem Elektropherogramm, die dem Proteinarzneimittel, Verunreinigungen und/oder Unreinheiten entsprechen, unter der Bedingung, dass die gepufferte Proteinarzneimittelprobe nicht für 5 bis 15 Minuten auf 65 bis 85 °C erhitzt wird, um die denaturierte gepufferte Proteinarzneimittelprobe zu bilden.

10. Verfahren nach Anspruch 9, wobei die gepufferte Proteinarzneimittelprobe 10 Minuten lang bei 60 °C erhitzt wird und/oder wobei die etikettierte Proteinarzneimittelprobe 15 Minuten lang bei 35 °C erhitzt wird.

11. Verfahren nach Anspruch 9, wobei die verdünnte Proteinarzneimittelprobe eine Konzentration von 3,6 µg/ml aufweist.

12. Verfahren nach einem der Ansprüche 9 bis 11, wobei das nachweisbare Etikett DY-631 N-Hydroxysuccinimidylester ist.

13. Verfahren nach einem der Ansprüche 9 bis 11, wobei das nachweisbare Etikett mit Dimethylsulfoxid (DMSO) rekonstituiert wird, gefolgt von einer Verdünnung mit 200 mM Natriumphosphatpuffer pH 7,2.

14. Kit, umfassend den Puffer nach einem der Ansprüche 1 bis 8, und schriftliche Anweisungen zum Vorbereiten einer Probe für die Elektrophorese im Puffer.

## Revendications

1. Tampon aqueux pour échantillon d'électrophorèse, comprenant :
155 à 200 mM de 2-iodoacétamide ;
0,50 à 1,5 % de dodécylsulfate de lithium ; et
60 à 95 mM de phosphate de sodium,
dans lequel le tampon aqueux pour échantillon d'électrophorèse présente un pH inférieur ou égal à 8,
à condition qu'il ne s'agisse pas d'un tampon aqueux pour échantillon d'électrophorèse ayant un pH inférieur à 7, et comprenant :
155 à 175 mM de 2-iodoacétamide ;
0,50 à 1,5 % de dodécylsulfate de lithium ; et
et 65 à 95 mM de phosphate de sodium,

2. Tampon aqueux selon la revendication 1, dans lequel le pH est de 6.

3. Tampon aqueux selon la revendication 1, comprenant 200 mM de 2-iodoacétamide, 1,2 % de dodécylsulfate de lithium, et 60 mM de phosphate de sodium.

4. Tampon aqueux pour échantillon d'électrophorèse selon la revendication 1, comprenant :
200 mM de 2-iodoacétamide,
1,2 % de dodécylsulfate de lithium, et
60 mM de phosphate de sodium,
dans lequel le tampon aqueux pour échantillon d'électrophorèse présente un pH de 6,0 ; ou comprenant :
200 mM de 2-iodoacétamide,
1,2 % de dodécylsulfate de lithium, et
60 mM de phosphate de sodium,
dans lequel le tampon aqueux pour échantillon d'électrophorèse présente un pH de 8,0.

5. Tampon aqueux pour échantillon d'électrophorèse, comprenant :
0,50 à 1,5 % de dodécylsulfate de lithium ;
45 à 75 mM de phosphate de sodium,
80 à 155 mM de dithiothréitol,
dans lequel le tampon aqueux pour échantillon d'électrophorèse présente un pH de 8 ou plus, par exemple dans lequel le pH est de 8 ou 9, et
à condition que le tampon aqueux pour échantillon d'électrophorèse ne comprenne pas 135 à 155 mM de dithiothréitol.

6. Tampon aqueux selon la revendication 5, comprenant 80 mM de dithiothréitol.

7. Tampon aqueux selon la revendication 5, comprenant en outre 1,2 % de dodécylsulfate de lithium et 60 mM de phosphate de sodium.

8. Tampon aqueux selon la revendication 5, consistant en :
0,69 % de dodécylsulfate de lithium ;
69 mM de phosphate de sodium, et
80 à 155 mM de dithiothréitol,
dans lequel le tampon aqueux pour échantillon d'électrophorèse présente un pH de 9,0 ; ou consistant en :
1,2 % de dodécyl sulfate de lithium ;
60 mM de phosphate de sodium, et
80 mM de dithiothréitol,
dans lequel le tampon aqueux pour échantillon d'électrophorèse présente un pH de 8,0.

9. Procédé d'identification de contaminants ou d'impuretés dans un échantillon de médicament protéique, le procédé comprenant les étapes suivantes :
l'ajout de l'échantillon de médicament protéique au tampon selon l'une quelconque des revendications 1 à 8 pour former un échantillon de médicament protéique tamponné ;
le chauffage de l'échantillon de médicament protéique tamponné à 45 à 75 °C pendant 5 à 15 minutes pour former un échantillon de médicament protéique tamponné dénaturé ;
l'ajout d'un marqueur détectable à l'échantillon de médicament protéique tamponné dénaturé et son chauffage à 30 à 40 °C pendant 10 à 40 minutes pour former un échantillon de médicament protéique dénaturé marqué ;
la dilution de l'échantillon de médicament protéique dénaturé marqué et sa soumission soit (a) à une électrophorèse capillaire sur micropuce (MCE) pour séparer l'échantillon de médicament protéique dilué sur un système d'électrophorèse capillaire à micropuce pour produire un électrophérogramme, soit (b) à une analyse MCE sur un système d'électrophorèse capillaire à micropuce pour produire un électrophérogramme ; et
l'identification de pics sur l'électrophérogramme correspondant au médicament protéique, aux contaminants et/ou aux impuretés,
à condition que l'échantillon de médicament protéique tamponné ne soit pas chauffé entre 65 et 85 °C pendant 5 à 15 minutes pour former l'échantillon de médicament protéique tamponné dénaturé.

10. Procédé selon la revendication 9, dans lequel l'échantillon de médicament protéique tamponné est chauffé à 60 °C pendant 10 minutes et/ou dans lequel l'échantillon de médicament protéique marqué est chauffé à 35 °C pendant 15 minutes.

11. Procédé selon la revendication 9, dans lequel l'échantillon de médicament protéique dilué présente une concentration de 3,6 µg/ml.

12. Procédé selon l'une quelconque des revendications 9 à 11, dans lequel le marqueur détectable est l'ester de N-hydroxysuccinimidyle DY-631.

13. Procédé selon l'une quelconque des revendications 9 à 11, dans lequel le marqueur détectable est reconstitué avec du diméthylsulfoxyde (DMSO) suivi d'une dilution avec un tampon au phosphate de sodium 200 mM pH 7,2.

14. Kit comprenant le tampon selon l'une quelconque des revendications 1 à 8, et
des instructions écrites pour préparer un échantillon pour une électrophorèse dans le tampon.
